# EUROPEAN PATENT APPLICATION

(11) **EP 1 977 772 A2**
(43) Date of publication of application: **08.10.2008**
(21) Application number: 08153460.4
(22) Date of filing: 27.03.2008
(51) Int. Cl.: A61L 31/16

(54) **Short term sustained drug-delivery system for implantable medical devices and method of making the same**

(30) Priority: 28.03.2007 US 692333
(71) Applicant: Cordis Corporation, Miami Lakes, FL 33014 (US)
(72) Inventor: Zhao, Jonathon Z., Belle Mead, NJ NJ 08502 (US)
(74) Representative: Belcher, Simon James

(57) **Abstract**

A short-term sustained drug eluting coating for implantable medical devices is disclosed. The coating comprises a biocompatible matrix and at least one pharmacologically or biologically active agent and releases substantially one of all of its payloads within four to six weeks post-implantation. When a combination of pharmacological agents are incorporated in the disclosed sustained drug eluting matrix of a drug/device combination, at least one agent is preferred to substantially release in a short duration. Medical devices benefiting from such a desired sustained drug eluting coating include drug eluting cardiovascular, peripheral, and neurovascular stents, abdominal aortic aneurysm (AAA) prosthesis, anastomosis shunt, arterial/venous (AV) shunts etc. One embodiment of the invention is a sustained release coating or depot on or in a stent that releases substantially all of it payload for ischemic myocardial injury after a heart attack. The coating may be formed from biocompatible stable and absorbable polymers of natural and synthetic origins. Useful pharmacological agents for inhibiting vascular neointimal growth post-angioplasty that leads to restenosis include macrolide polyenes such as a rapamycin and all its derivatives and analogs; paclitaxel and all derivatives and analogs. Useful agents for other vascular conditions such as vulnerable plaques may comprise anti-inflammatory, anti-proliferative agents, and matrix metalloprotease (MMP) inhibitors.

## Description

The present invention relates to sustained drug delivery systems for controlled and even short duration drug release from implantable medical devices. The drug delivery system comprises a biocompatible matrix, incorporating at least one pharmacologically and/or a biologically active compound, designed to release substantially all of the incorporated pharmacologically and/or biologically active compounds within zero to six weeks post-implantation and more preferably between two to six weeks post implantation. For a particular application such as reperfusion following a myocardial infarction, the sustained drug release during may be between one to two weeks. The biocompatible matrix may be bioabsorbable and be completely absorbed after the drug content is released. Also disclosed are methods of making such a coating and methods for treating local vascular diseases such as restenosis following angioplasty.

Controlled drug delivery is central to the success of the recently approved controlled delivery products such as Luprolide depots, and Nutropin depots. One class of drug device combination products that benefited immensely from a successfully designed controlled drug delivery system is the drug eluting stent (DES). The currently marketed products, including a sirolimus eluting coronary stent, Cypher, available from Cordis Corporation, a Johnson and Johnson Company, and a paclitaxel eluting stent, TAXUS, available from Boston Scientific Corporation, all employ a biostable polymeric coating to regulate the drug elution from the stent surface. A new generation DES such as the CoStar available from Conor Medsystems Inc. utilizes through wells or holes on the stent struts as drug delivery depots.

A stent is a type of endovascular implant, usually generally tubular in shape and having a lattice, connected element tubular construction that is expandable to be permanently inserted into a blood vessel to provide mechanical support to the vessel and to maintain or re-establish a flow channel during or following angioplasty procedure. The supporting structure of a stent, typically made of a metal or a metal alloy, is designed to counter the negative vessel remodeling process following an angioplasty. New stent designs such as the CoStar stents from Conor Medsystems also have holes or wells in the stent to enhance the stent □s ability to carry and release drug *in vivo.*

During the healing process of the vascular wall following a balloon angioplasty and/or a stent implantation, inflammation caused by angioplasty and stent implant injury often causes smooth muscle cell proliferation and regrowth inside the stent, thus potentially partially closing the flow channel, and thereby potentially reducing or eliminating the beneficial effect of the angioplasty/stenting procedure. This process is called restenosis. Healing of the damaged vessel and prevention of the neointimal growth resulting from the migration and proliferation of smooth muscle cells may last between two to four weeks. These pathological processes require intervention with a potent pharmacological agent such as a rapamycin or paclitaxel, often delivered through a supporting structure such as a drug eluting stent.

Blood clots may also potentially form inside of the newly implanted stent due to the minimal thrombotic nature of the stent surfaces or any coating applied on the surface of a stent. It is thus desirable to have immobilized slowly eluting anti-thrombotic agents such as heparin and cilostazol from the stent. A low level of drug elution is highly desirable in the initial period of stent implantation prior to full endothelialization of the implantable device. This process normally takes place in less than about two to six weeks, and is highly influenced by the composition of the surface of the implant, the nature of the drug loaded within or on the implant, and other factors. A separate pharmacologically and biologically active compound may be needed to combat the potential acute and sub-acute stent thromboses.

Stent coatings are known which contain bioactive agents that are designed to reduce or eliminate thrombosis or restenosis. Such bioactive agents may be dispersed or dissolved in either a bio-durable or bio-erodable polymer matrix that is attached to the surface of the stent elements prior to implant. After implantation, the bioactive agent diffuses out of the polymer matrix and preferably into the surrounding tissue over a varying length of time. If the polymer is bioerodable, in addition to release of the drug through the process of diffusion, the bioactive agent may also be released as the polymer degrades or dissolves, making the agent more readily available to the surrounding tissue environment. Bioerodable stents and biodurable stents that provide drug elution for a long period of time are known in the art.

Heparin, as well as other anti-platelet or anti-thrombolytic agents, that may be utilized as surface coatings are known and may be chemically bound to the surface of the stent to reduce thrombosis. A heparinized surface is known to interfere with the blood-clotting cascade in humans, preventing attachment of platelets (a precursor to thrombin) on the stent surface. Stents have been described which include both a heparin coated surface and an active agent stored inside of a coating. See, for example, US-6,231,600 and US-5,288,711.

More recently, rapamycin, an immunosuppressant reported to suppress both smooth muscle cell and endothelial cell growth, has been shown to have improved effectiveness against restenosis, when delivered from a polymer coating on a stent. See, for example, US-5,288, 711 and US-6,153, 252.

Conventional wisdom holds that a stent coating comprising a compound selected for inhibiting restenosis and similar diseases should provide a substantially continuous therapeutic dose for a minimum period of greater than four to eight weeks when released from a coating. It is thought that the compound should be effective, at a low dose, in inhibiting smooth muscle cell proliferation to cover such a long duration. Endothelial cells which line the inside surface of the vessel lumen are normally damaged by the process of angioplasty and/or stenting to some extent. A same or different compound should allow for a re-establishment of endothelial cells inside the vessel lumen, to provide a return to vessel homeostasis and to promote normal and critical interactions between the vessel walls and blood flowing through the vessel. This line of thinking has been translating into the design of the current generation of drug eluting stents. For example, the incorporated drug is released over a period of ninety days in the case of Cypher. In the case of TAXUS, after the initial burst of paclitaxel release, the drug continues to be released at a low dose for months. Most other current and next generation drug eluting stents all aim at providing drug release duration for a much longer duration beyond one month. More recently there are suggestions that late and very late stent thrombosis may be related to the long duration of a potent drug in the vascular wall.

On the other end of the spectrum, all incorporated drug may be released within the first week. The endeavor zotarolimus releasing stent from Medtronic has about sixty to seventy percent of loaded drug release within the first day post-implantation and substantially all of its pay-load within the first week. This fast and non-controlled drug release kinetics is attributed to the high late loss data observed in the clinical trials. Therefore, there exists a need to develop a well-designed drug delivery system that may deliver a drug load in a controlled and sustained fashion for a period of about zero to six weeks to adequately address all the pathological processes associated with an angioplasty procedure and/or stent implantation, while obviating the potential downside of negatively impacting the enthothelialization of the vessel by the lingering presence of a drug component.

In addition to the release duration of a therapeutic compound from a medical device, the amount of the drug release per unit, such as a day over the whole duration of drug release, from a medical device is equally important. This is not simply a case of dumping all drug content in a short time period, but rather an evenly controlled drug release per unit time such as a day. This is the short term release profile that is needed for the release duration for the scheme to work. For instance, after the initial burst of a drug from a drug-device combination product, an even and sustained drug release is highly desired and is positively correlated to the desired therapeutic efficacy of the drug for a given indication. For selected emerging applications such as interventional cardio-resuscitation post myocardial infarction, a relatively fast and even drug release from a drug device combination product is highly desired.

Therefore, there is a need to design a drug-device combination product that can produce a complete drug release within zero to six weeks, at an even unit dose per day for the entire duration after the initial burst.

The present invention overcomes the limitations associated with current devices as briefly described above.

In accordance with one aspect, the present invention is directed to a medical device configured for modulated drug release. The medical device comprises an implantable apparatus configured to release one or more bioactive agents over a period of time less than or equal to six weeks at a sustained and controlled rate per unit time. In various exemplary embodiments, a combination of pharmacologically active compounds includes a rapamycin and its analogs and derivatives, and an anti-thrombotic agent such as heparin and cilostazol. Exemplary anti-thrombotic agents may include: Vitamin K antagonist such as Acenocoumarol, Clorindione, Dicumarol, (Dicoumarol), Diphenadione, Ethyl biscoumacetate, Phenprocoumon, Phenindione, Tioclomarol, Warfarin; Heparin group anti-platelet aggregation inhibitors such as Antithrombin III, Bemiparin, Dalteparin, Danaparoid, Enoxaparin, Heparin, Nadroparin, Parnaparin, Reviparin, Sulodexide, Tinzaparin; other platelet aggregation inhibitors such as Abciximab, Acetylsalicylic acid (Aspirin), Aloxiprin, Beraprost, Ditazole, Carbasalate calcium, Cloricromen, Clopidogrel, Dipyridamole, Eptifibatide, Indobufen, Iloprost, Picotamide, Prasugrel, Prostacyclin, Ticlopidine, Tirofiban, Treprostinil, Trifusal; enzymatic anticoagulants such as Alteplase, Ancrod, Anistreplase, Brinase, Drotrecogin alfa, Fibrinolysin, Protein C, Reteplase, Saruplase, Streptokinase, Tenecteplase, Urokinase; direct thrombin inhibitors such as Argatroban, Bivalirudin, Dabigantran, Desirudin, Hirudin, Lepirudin, Melagatran, Ximelagatran; and other antithrombotics such as Dabigatran, Defibrotide, Dermatan sulfate, Fondaparinux, Rivaroxaban.

In other various exemplary embodiments, a myocardial beneficial agent includes a erythropoietin (EPO, Procrit), an insulin, and a morphine or morphine derivatives and analogs thereof, adenosine or adenosine analog or derivative thereof.

In yet other various exemplary embodiments, a pharmaceutically accepted additive such as the ones from Generally regarded as safe (GRAS) list, may be added to the coating matrix to speed up the drug release rate, and optionally the resorption time of the drug carrier matrix once the drug is exhausted.

The sustained drug delivery systems for controlled and even short duration drug release from implantable medical devices of the present invention are designed to deliver a drug load in a controlled and sustained fashion for a period ranging from about zero to about six weeks. In addition to the drug release duration, the periodic release rate or release rate over a given time period should also be controlled.

The sustained drug delivery systems for controlled and even short duration drug release from implantable medical devices of the present invention may be utilized to effectively prevent and treat vascular disease. Various medical treatment devices utilized in the treatment of vascular disease may ultimately induce further complications. For example, balloon angioplasty is a procedure utilized to increase blood flow through an artery and is the predominant treatment for coronary vessel stenosis. However, as stated above, the procedure typically causes a certain degree of damage to the vessel wall, thereby potentially exacerbating the problem at a point later in time. Although other procedures and diseases may cause similar injury, exemplary embodiments of the present invention will be described with respect to the treatment of restenosis and related complications following percutaneous transluminal coronary angioplasty and other similar arterial/venous procedures, including the joining of arteries, veins and other fluid carrying conduits.

While exemplary embodiments of the invention will be described with respect to the treatment of restenosis and related complications following percutaneous transluminal coronary angioplasty, it is important to note that the local delivery of drug/drug combinations may be utilized to treat a wide variety of conditions utilizing any number of medical devices, or to enhance the function and/or life of the device. For example, intraocular lenses, placed to restore vision after cataract surgery is often compromised by the formation of a secondary cataract. The latter is often a result of cellular overgrowth on the lens surface and can be potentially minimized by combining a drug or drugs with the device. Other medical devices which often fail due to tissue ingrowth or accumulation of proteinaceous material in, on and around the device, such as shunts for hydrocephalus, dialysis grafts, colostomy bag attachment devices, ear drainage tubes, leads for pace makers and implantable defibrillators can also benefit from the device-drug combination approach. Devices, which serve to improve the structure and function of tissue or organ, may also show benefits when combined with the appropriate agent or agents. For example, improved osteointegration of orthopedic devices to enhance stabilization of the implanted device could potentially be achieved by combining it with agents such as bone-morphogenic protein. Similarly other surgical devices, sutures, staples, anastomosis devices, vertebral disks, bone pins, suture anchors, hemostatic barriers, clamps, screws, plates, clips, vascular implants, tissue adhesives and sealants, tissue scaffolds, various types of dressings, bone substitutes, intraluminal devices, and vascular supports could also provide enhanced patient benefit using this drug-device combination approach. Perivascular wraps may be particularly advantageous, alone or in combination with other medical devices. The perivascular wraps may supply additional drugs to a treatment site. Essentially, any type of medical device may be coated in some fashion with a drug or drug combination, which enhances treatment over use of the singular use of the device or pharmaceutical agent.

In addition to various medical devices, the coatings on these devices may be used to deliver therapeutic and pharmaceutic agents including: anti-proliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) 11b/111a inhibitors and vitronectin receptor antagonists; anti-proliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes □dacarbazinine (DTIC); anti-proliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6□-methylprednisolone, triamcinolone, betamethasone, and dexamethasone), non-steroidal agents (salicylic acid derivatives i.e. aspirin; para-aminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; antisense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

As stated previously, the implantation of a coronary stent in conjunction with balloon angioplasty is highly effective in treating acute vessel closure and may reduce the risk of restenosis. Intravascular ultrasound studies (Mintz et al., 1996) suggest that coronary stenting effectively prevents vessel constriction and that most of the late luminal loss after stent implantation is due to plaque growth, probably related to neointimal hyperplasia. The late luminal loss after coronary stenting is almost two times higher than that observed after conventional balloon angioplasty. Thus, inasmuch as stents prevent at least a portion of the restenosis process, a combination of drugs, agents or compounds which prevents smooth muscle cell proliferation, reduces inflammation and reduces coagulation or prevents smooth muscle cell proliferation by multiple mechanisms, reduces inflammation and reduces coagulation combined with a stent may provide the most efficacious treatment for post-angioplasty restenosis. The systemic use of drugs, agents or compounds in combination with the local delivery of the same or different drug/drug combinations may also provide a beneficial treatment option.

The local delivery of drug/drug combinations from a stent has the following advantages; namely, the prevention of vessel recoil and remodeling through the scaffolding action of the stent and the prevention of multiple components of neointimal hyperplasia or restenosis as well as a reduction in inflammation and thrombosis. This local administration of drugs, agents or compounds to stented coronary arteries may also have additional therapeutic benefit. For example, higher tissue concentrations of the drugs, agents or compounds may be achieved utilizing local delivery, rather than systemic administration. In addition, reduced systemic toxicity may be achieved utilizing local delivery rather than systemic administration while maintaining higher tissue concentrations. Also in utilizing local delivery from a stent rather than systemic administration, a single procedure may suffice with better patient compliance. An additional benefit of combination drug, agent, and/or compound therapy may be to reduce the dose of each of the therapeutic drugs, agents or compounds, thereby limiting their toxicity, while still achieving a reduction in restenosis, inflammation and thrombosis. Local stent-based therapy is therefore a means of improving the therapeutic ratio (efficacy/toxicity) of anti-restenosis, anti-inflammatory, anti-thrombotic drugs, agents or compounds.

There are a multiplicity of different stents that may be utilized following percutaneous transluminal coronary angioplasty. Although any number of stents may be utilized in accordance with the present invention, for simplicity, a few stents will be described in exemplary embodiments of the present invention. The skilled artisan will recognize that any number of stents may be utilized in connection with the present invention. In addition, as stated above, other medical devices may be utilized.

A stent is commonly used as a tubular structure left inside the lumen of a duct to relieve an obstruction. Commonly, stents are inserted into the lumen in a non-expanded form and are then expanded autonomously, or with the aid of a second device in situ. A typical method of expansion occurs through the use of a catheter-mounted angioplasty balloon which is inflated within the stenosed vessel or body passageway in order to shear and disrupt the obstructions associated with the wall components of the vessel and to obtain an enlarged lumen.

Embodiments of the invention will now be described by way of example with reference to the accompanying drawings, in which:
Figure 1 is an isometric view of an expandable medical device with a beneficial agent at the ends in accordance with the present invention.
Figure 2 is an isometric view of an expandable medical device with a beneficial agent at a central portion and no beneficial agent at the ends in accordance with the present invention.
Figure 3 is an isometric view of an expandable medical device with different beneficial agents in different holes in accordance with the present invention.
Figure 4 is an isometric view of an expandable medical device with different beneficial agents in alternating holes in accordance with the present invention.
Figure 5 is an enlarged side view of a portion of an expandable medical device with beneficial agent openings in the bridging elements in accordance with the present invention.
Figure 6 is an enlarged side view of a portion of an expandable medical device with a bifurcation opening in accordance with the present invention.

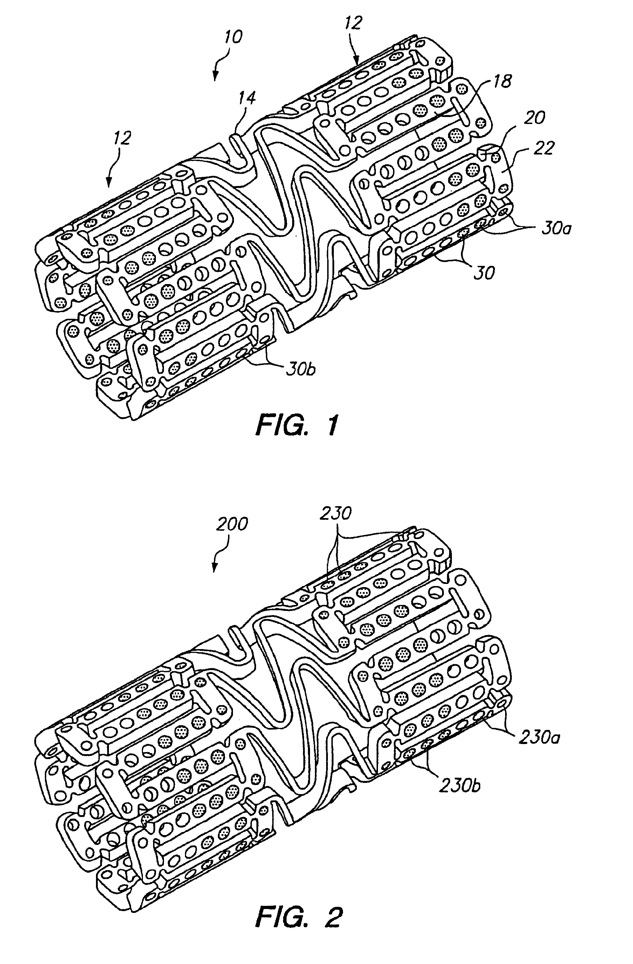

Figure 1 illustrates an expandable medical device having a plurality of holes containing a beneficial agent for delivery to tissue by the expandable medical device. The expandable medical device 100 illustrated in Figure 1 is cut from a tube of material to form a cylindrical expandable device. The expandable medical device 100 includes a plurality of cylindrical sections 102 interconnected by a plurality of bridging elements 104. The bridging elements 104 allow the tissue supporting device to bend axially when passing through the torturous path of vasculature to a deployment site and allow the device to bend axially when necessary to match the curvature of a lumen to be supported. Each of the cylindrical tubes 102 is formed by a network of elongated struts 108 which are interconnected by ductile hinges 110 and circumferential struts 112. During expansion of the medical device 100 the ductile hinges 110 deform while the struts 108 are not deformed. Further details of one example of the expandable medical device are described US-6,241,762.

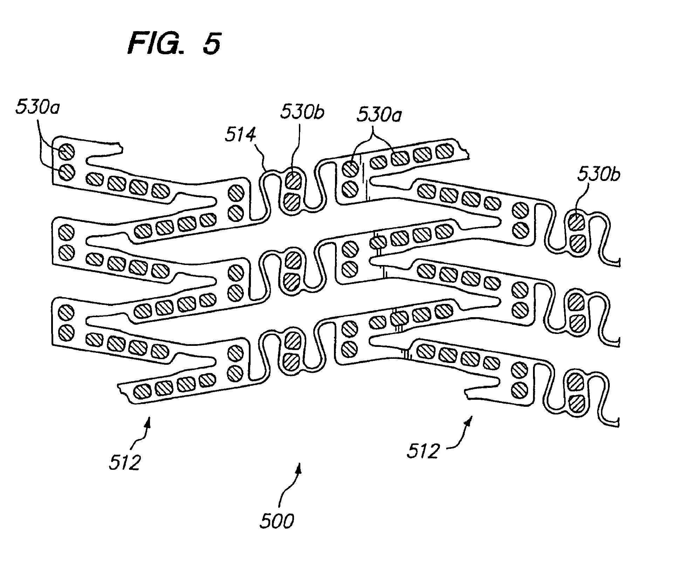

As illustrated in Figure 1, the elongated struts 108 and circumferential struts 112 include openings 114, some of which contain a beneficial agent for delivery to the lumen in which the expandable medical device is implanted. In addition, other portions of the device 100, such as the bridging elements 104, may include openings, as discussed below with respect to Figure 5. Preferably, the openings 114 are provided in non-deforming portions of the device 100, such as the struts 108, so that the openings are non-deforming and the beneficial agent is delivered without risk of being fractured, expelled, or otherwise damaged during expansion of the device. A further description of one example of the manner in which the beneficial agent may be loaded within the openings 114 is described in U.S. patent application Serial No. 09/948,987, filed Sep. 7, 2001, published as US-A-2002/0068969.

The exemplary embodiments of the present invention illustrated may be further refined by using Finite Element Analysis and other techniques to optimize the deployment of the beneficial agents within the openings 114. Basically, the shape and location of the openings 114, may be modified to maximize the volume of the voids while preserving the relatively high strength and rigidity of the struts with respect to the ductile hinges 110. According to one preferred exemplary embodiment of the present invention, the openings have an area of at least 0.0032 mm² (5 x 10⁻⁶ square inches), and preferably at least 0.0045 mm² (7 x 10⁻⁶ square inches). Typically, the openings are filled about 50 percent to about 95 percent full of beneficial agent.

Figure 1 illustrates an expandable medical device 100 with "hot ends" or beneficial agent provided in the openings 114 at the ends of the device in order to treat and reduce edge effect restenosis. The remaining openings 114 in the central portion of the device may be empty (as shown) or may contain a lower concentration of beneficial agent.

Figure 2 illustrates an alternate exemplary embodiment of an expandable medical device 200 having a plurality of openings 230 in which the openings 230b in a central portion of the device are filled with a beneficial agent and the openings 230a at the edges of the device remain empty. The device of Figure 2 is referred to as having "cool ends."

In addition to use in reducing edge effect restenosis, the expandable medical device 200 of Figure 2 may be used in conjunction with the expandable medical device 100 of Figure 1 or another drug delivery stent when an initial stenting procedure has to be supplemented with an additional stent. For example, in some cases the device 100 of Figure 1 with "hot ends" or a device with uniform distribution of drug may be implanted improperly. If the physician determines that the device does not cover a sufficient portion of the lumen a supplemental device may be added at one end of the existing device and slightly overlapping the existing device. When the supplemental device is implanted, the device 200 of Figure 2 is used so that the "cool ends" of the medical device 200 prevent double-dosing of the beneficial agent at the overlapping portions of the devices 100, 200.

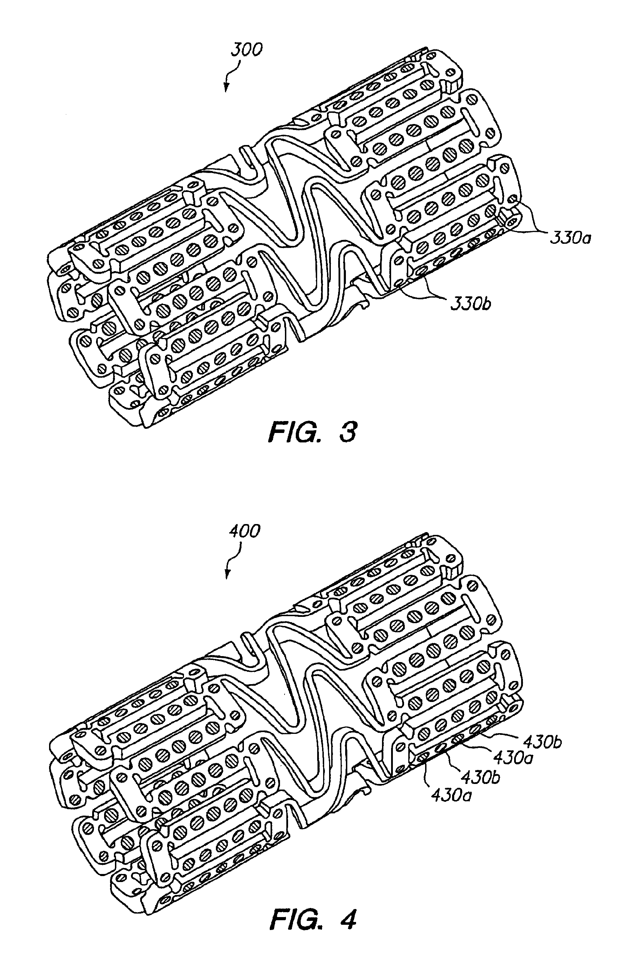

Figure 3 illustrates a further alternate exemplary embodiment of the invention in which different beneficial agents are positioned in different holes of an expandable medical device 300. A first beneficial agent is provided in holes 330a at the ends of the device and a second beneficial agent is provided in holes 330b at a central portion of the device. The beneficial agent may contain different drugs, the same drugs in different concentrations, or different variations of the same drug. The exemplary embodiment of Figure 3 may be used to provide an expandable medical device 300 with either "hot ends" or "cool ends."

Preferably, each end portion of the device 300 which includes the holes 330a comprising the first beneficial agent extends at least one hole and up to about 15 holes from the edge. This distance corresponds to about 0.13 mm (0.005 inches) to about 2.5 mm (0.1 inches) from the edge of an unexpanded device. The distance from the edge of the device 300 which includes the first beneficial agent is preferably about one section, where a section is defined between the bridging elements.

Different beneficial agents comprising different drugs may be disposed in different openings in the stent. This allows the delivery of two or more beneficial agents from a single stent in any desired delivery pattern. Alternately, different beneficial agents comprising the same drug in different concentrations may be disposed in different openings. This allows the drug to be uniformly distributed to the tissue with a non-uniform device structure.

The two or more different beneficial agents provided in the devices described herein may comprise (1) different drugs; (2) different concentrations of the same drug; (3) the same drug with different release kinetics, e.g., different matrix erosion rates; or (4) different forms of the same drug. Examples of different beneficial agents formulated comprising the same drug with different release kinetics may use different carriers to achieve the elution profiles of different shapes. Some examples of different forms of the same drug include forms of a drug having varying hydrophilicity or lipophilicity.

In one example of the device 300 of Figure 3, the holes 330a at the ends of the device are loaded with a first beneficial agent comprising a drug with a high lipophilicity while holes 330b at a central portion of the device are loaded with a second beneficial agent comprising the drug with a lower lipophilicity. The first high lipophilicity beneficial agent at the "hot ends" will diffuse more readily into the surrounding tissue reducing the edge effect restenosis.

The device 300 may have an abrupt transition line at which the beneficial agent changes from a first agent to a second agent. For example, all openings within 1.3 mm (0.05 inches) of the end of the device may comprise the first agent while the remaining openings comprise the second agent. Alternatively, the device may have a gradual transition between the first agent and the second agent. For example, a concentration of the drug in the openings may progressively increase (or decrease) toward the ends of the device. In another example, an amount of a first drug in the openings increases while an amount of a second drug in the openings decreases moving toward the ends of the device.

Figure 4 illustrates a further alternate exemplary embodiment of an expandable medical device 400 in which different beneficial agents are positioned in different openings 430a, 430b in the device in an alternating or interspersed manner. In this manner, multiple beneficial agents may be delivered to tissue over the entire area or a portion of the area supported by the device. This exemplary embodiment will be useful for delivery of multiple beneficial agents where combination of the multiple agents into a single composition for loading in the device is not possible due to interactions or stability problems between the beneficial agents.

In addition to the use of different beneficial agents in different openings to achieve different drug concentrations at different defined areas of tissue, the loading of different beneficial agents in different openings may be used to provide a more even spatial distribution of the beneficial agent delivered in instances where the expandable medical device has a non-uniform distribution of openings in the expanded configuration.

The use of different drugs in different openings in an interspersed or alternating manner allows the delivery of two different drugs which may not be deliverable if combined within the same polymer/drug matrix composition. For example, the drugs themselves may interact in an undesirable way. Alternatively, the two drugs may not be compatible with the same polymers for formation of the matrix or with the same solvents for delivery of the polymer/drug matrix into the openings.

Further, the exemplary embodiment of Figure 4 having different drugs in different openings in an interspersed arrangement provide the ability to deliver different drugs with very different desired release kinetics from the same medical device or stent and to optimize the release kinetic depending on the mechanism of action and properties of the individual agents. For example, the water solubility of an agent greatly affects the release of the agent from a polymer or other matrix. A highly water soluble compound will generally be delivered very quickly from a polymer matrix, whereas, a lipophilic agent will be delivered over a longer time period from the same matrix. Thus, if a hydrophilic agent and a lipophilic agent are to be delivered as a dual drug combination from a medical device, it is difficult to achieve a desired release profile for these two agents delivered from the same polymer matrix.

The system of Figure 4 allows the delivery of a hydrophilic and a lipophilic drug easily from the same stent. Further, the system of FIG. 4 allows the delivery two agents at two different release kinetics and/or administration periods. Each of the initial release in the first twenty-four hours, the release rate following the first twenty-four hours, the total administration period and any other characteristics of the release of the two drugs may be independently controlled. For example the release rate of the first beneficial agent may be arranged to be delivered with at least forty percent (preferably at least fifty percent) of the drug delivered in the first twenty-four hours and the second beneficial agent may be arranged to be delivered with less than twenty percent (preferably less than ten percent) of the drug delivered in the first twenty-four hours. The administration period of the first beneficial agent may be about three weeks or less (preferably two weeks or less) and the administration period of the second beneficial agent may be about four weeks or more.

Restenosis or the recurrence of occlusion post-intervention, involves a combination or series of biological processes. These processes include the activation of platelets and macrophages. Cytokines and growth factors contribute to smooth muscle cell proliferation and up regulation of genes and metalloproteinases lead to cell growth, remodeling of extracellular matrix, and smooth muscle cell migration. A drug therapy which addresses a plurality of these processes by a combination of drugs may be the most successfully antirestenotic therapy. The present invention provides a means to achieve such a successful combination drug therapy.

One example of a beneficial system for delivering two drugs from interspersed or alternating holes is the delivery of an anti-inflammatory agent or an immunosuppressant agent in combination with an antiproliferative agent or an anti-migratory agent. Other combinations of these agents may also be used to target multiple biological processes involved in restenosis. The anti-inflammatory agent mitigates the initial inflammatory response of the vessel to the angioplasty and stenting and is delivered at a high rate initially followed by a slower delivery over a time period of about two weeks to match the peak in the development of macrophages which stimulate the inflammatory response. The antiproliferative agent is delivered at a relatively even rate over a longer time period to reduce smooth muscle cell migration and proliferation.

The following chart illustrates some of the useful two drug combination therapies which may be achieved by placing the drugs into different openings in the medical device.

| | PTX2-Cda | Epothilone D | Imatinibmesylate Gleevec | Rapamycin analog | Pimecrolimus | PKC-412 | Dexamethasone | Farglitazar | Insulin | VIP | ApoA-I milano |
|---|---|---|---|---|---|---|---|---|---|---|---|
| PTX | x | | x | | x | x | x | | x | x | x |
| 2-CdA | | x | x | x | x | x | | s | | | |
| Epothilone D | | | x | | | x | x | | x | x | x |
| Imatinib | | | | x | x | x | x | | | | |
| mesylate | | | | | | | | | | | |
| Gleevec | | | | | | | | | | | |
| Rapamycin | | | | | | x | x | | x | x | x |
| analog | | | | | | | | | | | |
| Pimecrolimus | | | | | | x | x | | x | x | x |
| PKC-412 | | | | | | | x | | x | x | x |
| Dexamethasone | | | | | | | | | | x | x |
| Farglitazar | | | | | | | | | | x | x |
| Insulin | | | | | | | | | | x | |
| VIP | | | | | | | | | | | x |
| ApoA-1 milano | | | | | | | | | | | |

The placement of the drugs in different openings allows the release kinetics to be tailored to the particular agent regardless of the hydrophobilicity or lipophobicity of the drug. Examples of some arrangements for delivery of a lipophilic drug at a substantially constant or linear release rate are described in WO 04/110302 published on December 23, 2004. Examples of some of the arrangements for delivery of hydrophilic drug are described in WO 04/043510, published on May 27, 2004. The hydrophilic drugs listed above include CdA, Gleevec, VIP, insulin, and ApoA-1 milano. The lipophilic drugs listed above include paclitaxel, Epothilone D, rapamycin, pimecrolimus, PKC-412 and Dexamethazone. Farglitazar is partly liphophillic and partly hydrophilic.

In addition to the delivery of multiple of drugs to address different biological processes involved in restenosis, the present invention may deliver two different drugs for treatment of different diseases from the same stent. For example, a stent may deliver an anti-proliferative, such as paclitaxel or a limus drug from one set of openings for treatment of restenosis while delivering a myocardial preservative drug, such as insulin, from other openings for the treatment of acute myocardial infarction.

In many of the known expandable devices and for the device illustrated in Figure 5 the coverage of the device 500 is greater at the cylindrical tube portions 512 of the device than at the bridging elements 514. Coverage is defined as the ratio of the device surface area to the area of the lumen in which the device is deployed. When a device with varying coverage is used to deliver a beneficial agent contained in openings in the device, the beneficial agent concentration delivered to the tissue adjacent the cylindrical tube portions 512 is greater that the beneficial agent delivered to the tissue adjacent the bridging elements 514. In order to address this longitudinal variation in device structure and other variations in device coverage which lead to uneven beneficial agent delivery concentrations, the concentration of the beneficial agent may be varied in the openings at portions of the device to achieve a more even distribution of the beneficial agent throughout the tissue. In the case of the exemplary embodiment illustrated in Figure 5, the openings 530a in the tube portions 512 include a beneficial agent with a lower drug concentration than the openings 530b in the bridging elements 514. The uniformity of agent delivery may be achieved in a variety of manners including varying the drug concentration, the opening diameter or shape, the amount of agent in the opening (i.e., the percentage of the opening filed), the matrix material, or the form of the drug.

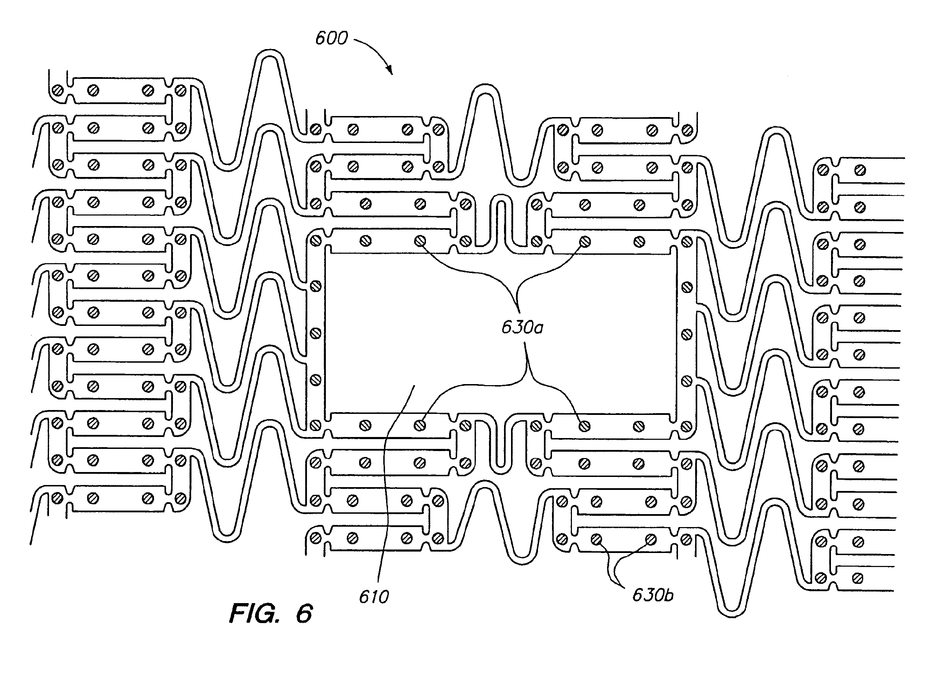

Another example of an application for the use of different beneficial agents in different openings is in an expandable medical device 600, as illustrated in Figure 6, configured for use at a bifurcation in a vessel. Bifurcation devices include a side hole 610 which is positioned to allow blood flow through a side branch of a vessel. One example of a bifurcation device is described in US-6,293,967. The bifurcation device 600 includes the side hole feature 610 interrupting the regular pattern of beams which form a remainder of the device. Since an area around a bifurcation is a particularly problematic area for restenosis, a concentration of an antiproliferative drug may be increased in openings 830a at an area surrounding the side hole 610 of the device 600 to deliver increased concentrations of the drug where needed. The remaining openings 630b in an area away from the side opening contain a beneficial agent with a lower concentration of the antiproliferative. The increased antiproliferative delivered to the region surrounding the bifurcation hole may be provided by a different beneficial agent containing a different drug or a different beneficial agent containing a higher concentration of the same drug.

In addition to the delivery of different beneficial agents to the mural or abluminal side of the expandable medical device for treatment of the vessel wall, beneficial agents may be delivered to the luminal side of the expandable medical device to prevent or reduce thrombosis. Drugs which are delivered into the blood stream or contact the blood stream from the luminal side of the device may be located at a proximal end of the device or a distal end of the device.

The methods for loading different beneficial agents into different openings in an expandable medical device may include known techniques such as dipping and coating and also known piezoelectric micro-jetting techniques. Micro-injection devices may be computer controlled to deliver precise amounts of two or more liquid beneficial agents to precise locations on the expandable medical device in a known manner. For example, a dual agent jetting device may deliver two agents simultaneously or sequentially into the openings. When the beneficial agents are loaded into through openings in the expandable medical device, a luminal side of the through openings may be blocked during loading by a resilient mandrel allowing the beneficial agents to be delivered in liquid form, such as with a solvent. The beneficial agents may also be loaded by manual injection devices.

Although the exemplary embodiments of stents described above utilize holes or wells, the drugs are typically incorporated in polymeric matrices or vehicles and then in the holes or wells. The principles described below with respect to elution control are applicable to the stent concepts described above or to surface coatings as described below. In other words, the techniques for controlling drug elution described herein are applicable to any stent design regardless of how the drug/polymer combination is applied.

The polymer forming the substrate or matrix into which the drug or drugs is incorporated may be any biocompatible polymer material from which entrapped compound may be released by diffusion and/or released by erosion of the polymer matrix.

Bioerodable polymers, particularly copolymers of poly(d,1-lactide-co-glycolide) (PLGA), are especially suitable as a coating substrate material. In one exemplary embodiment, of the invention, the coating is a bioerodable PLGA with a molar ratio of LA and GA between 80:20 to 20:80. PLGA with close to 50:50 molar being the more preferred polymers, may provide a hydrophobic matrix for sustained drug release and a relatively short residence time. It is known in the arts that PLGA 50:50 have the shortest degradation time, given the same molecular weight.

In accordance with the present invention, the drug loading level may be from ten percent to ninety percent, depending on the required release duration for the intended application. A higher drug loading level generally leads to a faster release rate, and consequently a shorter drug action time.

Another way to modulate the drug release duration from the coating is through changing it physical form. For instance, it is easier to make an amorphous form of a drug to form a physical solution with its carrier matrix.

One preferred coating is formed of 20%-75% (wt) polymer substrate, and 80%-25% (wt) of a rapamycin as a device coating.

The coating may additionally include a second bioactive agent effective to minimize blood-related events, such as clotting, that may be stimulated by the original vascular injury, the presence of the stent, or to improve vascular healing at the injury site. Exemplary second agents include anti-platelet, fibrinolytic, or thrombolytic agents in soluble crystalline form. Exemplary anti-platelet, fibrinolytic, or thrombolytic agents are heparin, aspirin, hirudin, ticlopadine(sp), eptifibatide, urokinase, streptokinase, tissue plasminogen activator (tPA), or mixtures thereof. The amount of second agent included in the stent coating will be determined by the period over which the agent will need to provide therapeutic benefit. Typically, the agent will be beneficial over the first two weeks after vascular injury and stent implantation, although for some agents, longer period of release of the agent will be more advantageous.

In another exemplary embodiment, both the stent body and polymer coating are formed of a bioerodable polymer, allowing complete resorption of the stent over time. The coating polymer may be the same or different from the polymer used to construct the stent scaffold. The coating polymer in normal situations should have a lower molecular weight and a comparatively less crystalline form, allowing for better solubility in common organic solvents and faster drying time.

Methods for forming balloon-expandable stents formed of a knitted, bioerodable polymer filament such as poly-1-lactide have been reported (US-6,080,177). A version of the device has also been adapted to release drugs (US-5,733,327).

A preferred polymer material for forming the stent is poly(1-lactide (PLLA). As indicated above, the stent body and coating may be formed integrally as a single expandable filament stent having anti-restenosis compound contained throughout. Alternately, a bioerodable coating may be applied to a preformed bioerodable body, as detailed below. In the latter case, the stent body may be formed of one bioerodable polymer, such as PLLA, and the coating from a second polymer, such as PLGA polymer. The coating, if applied to a preformed stent, may have substantially the same compositional and thickness characteristics described above.

The bioerodable stent has the unique advantage of treating the entire vessel with one device, either in conjunction with pre-dilitation of the vessel with balloon angioplasty if large obstructions are present, or as a prophylactic implant in patients of high risk of developing significant future blockages. Since the stent is fully biodegradeable, it does not affect the patient's chances for later uncomplicated surgery on the vessel, as a "full metal jacket," i.e., a string of drug eluting stents containing metal substrates, does.

A secondary agent, such as indicated above, may be incorporated into the coating for release from the coating over a desired time period after implantation. Alternately, if a secondary agent is used, it may be incorporated into the stent-body elements if the coating applied to the stent body does not cover the interior surfaces of the stent body. The coating methods described below with respect to a metal stent body are also suitable for use in coating a polymer- filament stent body.

Typically, the coating is applied directly onto the outside support surface(s) of a stent, and may or may not cover the entire or a portion(s) of the inside surface(s) of the stent depending on how control is applied to the above described coating system of the present invention. Alternately, the coating or coating mixture can also be applied directly onto the inside surface of the stent. A thin delivery tip may penetrate through one or more of the cut out areas (i.e. windows) in the wall of the stent structure, and thereby apply the coating mixture directly onto the inside surfaces at desired areas. In this method, it is possible to apply different coating materials having different drug components to outer and inner sides of the stent elements. For example, the coating on the outer surfaces could contain an anti-restenosis compound, and the coating of the inner surfaces, one of the above secondary agents, such an anti-thrombotic or anti-clotting compound. If the stent has a large enough diameter, a thin "L- shaped" delivery tip may be inserted into the stent open ends along the longitudinal axis of the stent for the purpose of applying coating to the inside surfaces.

The polymer for use in the invention includes, a polymer, or a combination of polymers, dissolved in the first fluid. The polymeric material is most suitably biocompatible, including polymers that are non-toxic, non-inflammatory, chemically inert, and substantially non-immunogenic in the applied amounts. The polymer is typically either bioabsorbable or biostable. A bioabsorbable polymer breaks down in the body and is not present sufficiently long after implantation to cause an adverse local response. Bioabsorbable polymers are gradually absorbed or eliminated by the body by hydrolysis, metabolic process, bulk, or surface erosion. Examples of bioabsorbable materials include polycaprolactone (PCL), poly-D, L-lactic acid (DL-PLA), poly-L-lactic acid (L-PLA), poly(lactide-co-glycolide), poly(hydroxybutyrate), poly(hydroxybutyrate-co-valerate), polydioxanone, polyorthoester, polyanhydride, poly(glycolic acid), poly(glycolic acid-cotrimethylene carbonate), polyphosphoester, polyphosphoester urethane, poly (amino acids), cyanoacrylates, poly(trimethylene carbonate), poly(iminocarbonate), copoly(ether-esters), polyalkylene oxalates, polyphosphazenes, polyiminocarbonates, and aliphatic polycarbonates. Biomolecules such as heparin, fibrin, fibrinogen, cellulose, starch, and collagen are typically also suitable. A biostable polymer include Parylene®, Parylast®, polyurethane (for example, segmented polyurethanes such as Biospan®), polyethylene, polyethlyene teraphthalate, ethylene vinyl acetate, silicone, polyethylene oxide, and polytetrafluoroethylene (PTFE), polyacrylates and polymethacrylates.

Another preferred embodiment of the current invention may include a porous surface of the device, and having drug loaded therein, and optionally having a thin layer of polymer overcoat to regulate the drug release. A pharmaceutically acceptable excipient such as a mono-,di-, or oligo-saccharide may be included in the drug mixture to modulate the drug release. a micronized heparin can also be used a release regulator. As an example, heparin in crystalline form may be incorporated into the coating. The heparin crystals are micronized to a particle size of approximately one to five microns and added in suspension to the polymer solution. Suitable forms of heparin are those of crystalline form that exhibit bioactivity in mammalian hosts when applied according to the process of the invention, including heparin salts (i.e. sodium heparin and low molecular weight forms of heparin and their salts). Upon deployment of the drug delivering stent into the vessel wall, the heparin crystals near the surface of the coating begin to dissolve, increasing the porosity of the polymer. As the polymer slowly dissolves, more heparin and bioactive agent are released in a controlled manner.

A controlled drug delivery system in accordance with the present invention may be associated with an implantable medical device in a physical or chemical way. For instance, the controlled drug delivery system may be surface coating on a bare metal stent. Such coatings may gradually and evenly release the drug payload over a period of zero to six weeks upon implantation. After the commonly seen initial drug release burst of less than thirty percent in the first couple of days, the remaining drug is released over the next several weeks. The ideal amount of drug released per day varies with the potency and the toxicity of the drug. The commonly used measure is the therapeutic index of a drug. The therapeutic index, also known as therapeutic ratio or margin of safety, is a comparison of the amount of a therapeutic agent that causes the therapeutic effect to the amount that causes toxic effects. Quantitatively, it is the ratio given by the dose required to produce the toxic effect divided by the therapeutic dose. A commonly used measure of therapeutic index is the lethal dose of a drug for 50percent of the population (LD₅₀) divided by the effective dose for 50percent of the population (ED₅₀). For a drug with a larger therapeutic ratio such as a rapamycin, the permitted daily dose would be equal to or less than 3 ug/day for a coronary drug eluting stent in human after the intial drug burst. On the other hand, a more toxic drug such as paclitaxel, the therapeutic window is much narrower. It is preferred to be less than 0.5 ug/day for a coronary drug eluting stent.

Exemplary procedures that may be used to modulate the release kinetics of a drug from a carrier matrix on or in a medical device are given below. Post drug loading solvent treatment. This method may be used to change physical properties of a polymer carrier matrix such that the outermost layer/s become denser and less permeable to the penetration of the water or physiological medium into the matrix. Likely mechanisms include a dense packing of the matrix, increased crystallinity of matrix due to better orientation of the polymer matrix, and increased glass transition temperature (Tg) after the solvent. The solvent used needs to be able to briefly soften and/or dissolve the polymer/drug matrix before the removal of the solvent.

Another exemplary way to modulate the drug release from the drug eluting medical device is through the charge interactions of the matrix used to load the drug in. For instance, a cationic polymer species may be used as one layer of matrix followed by a layer of anionic polymer matrix. Such an arrangement may lead to a dense barrier layer to effectively modulate the drug release.
Another exemplary way to modulate the drug release from the drug eluting medical device is through the used of an osmotically active species to speed up the water uptake once the drug/device is implanted *in vivo.* These osmotically active species may imbibe the water and flush out the loaded drug so one hundred percent of drug load may be released within zero to six week *in vivo.* The percentage of osmotic species may be adjusted to precisely modulate the drug release rate.

Another exemplary way to modulate the drug release from the drug eluting medical device is through the use of the same polymer with different molecular weight (mw) in different layers. For instance, a low molecular weight polymer generally leads to faster release of drug from the matrix, while a higher molecular weight polymer leads to a comparatively slower rate.

Another exemplary way to modulate the drug release from the drug eluting medical device is through the use of polymer blends of various physical and/or chemical properties to help precisely modulate the drug elution. For instance, the more crystalline polymer in the blend tends to prolong the release duration of the drug.

Another exemplary way to modulate the drug release from the drug eluting medical device is through the use of combination of drugs. For instance, a hydrophilic drug when combined with a more hydrophobic drug will serve to speed up the release rata of the hydrophobic drug. For instance, when Cladribine is combined with a sirolimus in the same layer, the release rate of sirolimus may be sufficiently sped up to have one hundred percent within zero to six weeks *in vivo.* The molar of Cladribine in the drug combination may be used to further precisely modulate the sirolimus release rate.

Another exemplary way to modulate the drug release from the drug eluting medical device is through the use of pharmaceutically acceptable excipients such as mannitol, dextran, trehelose etc. These hydrophilic excipient help absorb water into the drug matrix to speed up the drug release. Similarly a comparative more hydrophobic excipient such as vitamin E may be used to modulate the drug release to a comparatively slower rate.

Another exemplary way to modulate the drug release from the drug eluting medical device is through the use of an amphiphilic polymer. These polymers may be tailored to present a given range of overall hydrophilic profile that is more conducive to further drug release rate. One example is a poly(lactide)-poly(ethylene glycol)-poly(lactide) terpolymer.

Another exemplary way to modulate the drug release from the drug eluting medical device is through the use of drug microsphere or nanosphere before they are deposited onto or into a medical device. These formulated drug containing microsphere or nanosphere may be affixed to the surface of a medical device or into wells or indentation of a medical device. Additional layer/s of polymers may be used to further regulate the drug release.

These methods are not meant to be exhaustive. One ordinarily skilled in the art would find similar ways to achieve similar level of drug release from a drug containing medical device without deviating from the scope of the present invention.

## Claims

1. A medical device configured for modulated drug release comprising an implantable apparatus configured to release one or more bioactive agents over a period of time less than or equal to six weeks at a sustained and controlled rate per unit time.

2. The medical device configured for modulated drug release according to claim 1, wherein the implantable apparatus comprises a stent.

3. The medical device configured for modulated drug release according to claim 2, wherein the stent is fabricated from a metallic material.

4. The medical device configured for modulated drug release according to claim 3, wherein the implantable apparatus further comprises a polymeric matrix for modulating the release of the one or more bioactive agents, the polymeric matrix being affixed to at least a portion of the stent.

5. The medical device configured for modulated drug release according to claim 3, wherein the surface of the stent comprises a structure for modulating the release of the one or more bioactive agents.

6. The medical device configured for modulated drug release according to claim 3, wherein the implantable apparatus further comprises a polymeric matrix for holding the one or more bioactive agents, the one or more bioactive agents comprising over fifty percent, by weight, of the polymeric matrix and the one or more biologically active agent combination.

7. The medical device configured for modulated drug release according to claim 2, wherein the stent is fabricated from a polymeric material.

8. The medical device configured for modulated drug release according to claim 7, wherein the polymeric material is biodegradable.

9. The medical device configured for modulated drug release according to claim 7, wherein the polymeric material is non-biodegradable.

10. The medical device configured for modulated drug release according to claim 7, wherein the implantable apparatus further comprises a polymeric matrix for modulating the release of the one or more bioactive agents, the polymeric matrix being affixed to at least a portion of the stent.

11. The medical device configured for modulated drug release according to claim 7, wherein the surface of the stent comprises a structure for modulating the release of the one or more bioactive agents.

12. The medical device configured for modulated drug release according to claim 7, wherein the implantable apparatus further comprises a polymeric matrix for holding the one or more bioactive agents, the one or more bioactive agents comprising over fifty percent, by weight, of the polymeric matrix and the one or more biologically active agent combination.

13. The medical device configured for modulated drug release according to claim 4, wherein the polymeric matrix further comprises a blend of polymers of varying hydrophilicity to effectively modulate the drug release to less than six weeks *in vivo.*

14. The medical device configured for modulated drug release according to claim 4 further comprises a blend of polymers of varying crystallinity to effectively modulate the drug release to less than six weeks *in vivo.*

15. The medical device configured for modulated drug release according to claim 4 further comprises polymers of varying ionic charges to effectively modulate the drug release to less than six weeks *in vivo.*

16. The medical device configured for modulated drug release according to claim 4 is further treated by a solvent to effectively modulate the drug release to less than six weeks *in vivo.*

17. The medical device configured for modulated drug release according to claim 4 further comprises a pharmaceutically acceptable excipient to effectively modulate the drug release to less than six weeks *in vivo.*

18. The medical device configured for modulated drug release according to claim 4 further comprises polymeric micro particles containing the one or more drug to effectively modulate the drug release to less than six weeks *in vivo.*

19. The medical device configured for modulated drug release according to claim 4 further comprises polymeric nanoparticles containing the one or more drug to effectively module the drug release to less than six weeks *in vivo.*

20. The medical device configured for modulated drug release according to claim 4 further comprises an amphiphilic polymer to effectively modulate the drug release to less than six weeks *in vivo.*

21. The medical device configured for modulated drug release according to claim 4 further comprises an osmotically active excipient to effectively modulate the drug release to less than six weeks *in vivo.*
